(19) Europäisches Patentamt
European Patent Office
Office européen des brevets

(11) **EP 2 654 420 B1**

(12) **EUROPÄISCHE PATENTSCHRIFT**

(45) Veröffentlichungstag und Bekanntmachung des Hinweises auf die Patenterteilung:
**19.10.2016 Patentblatt 2016/42**

(21) Anmeldenummer: **11804958.4**

(22) Anmeldetag: **16.12.2011**

(51) Int Cl.:
*A01N 25/04* (2006.01)      *A01N 25/32* (2006.01)
*A01N 31/02* (2006.01)      *A61K 8/31* (2006.01)
*A61K 8/34* (2006.01)      *A61K 8/37* (2006.01)
*A61K 8/81* (2006.01)      *A61K 8/893* (2006.01)
*A61K 31/045* (2006.01)      *A61Q 17/00* (2006.01)
*A61Q 19/00* (2006.01)      *A61Q 19/10* (2006.01)

(86) Internationale Anmeldenummer:
**PCT/EP2011/006382**

(87) Internationale Veröffentlichungsnummer:
**WO 2012/084164 (28.06.2012 Gazette 2012/26)**

(54) **ANTIMIKROBIELLE ZUSAMMENSETZUNG MIT HAUTPFLEGENDEN EIGENSCHAFTEN**

ANTIMICROBIAL COMPOSITION HAVING SKIN CARE PROPERTIES

COMPOSITION ANTIMICROBIENNE PRÉSENTANT DES PROPRIÉTÉS DE SOINS CUTANÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2010 DE 102010055528**

(43) Veröffentlichungstag der Anmeldung:
**30.10.2013 Patentblatt 2013/44**

(73) Patentinhaber: **Bode Chemie GmbH 22525 Hamburg (DE)**

(72) Erfinder:
• **HURTMANNS, Stephan 22455 Hamburg (DE)**
• **EGGERSTEDT, Sven 22527 Hamburg (DE)**

(74) Vertreter: **Königer, Karsten et al Harmsen Utescher Rechtsanwälte Patentanwälte Neuer Wall 80 20354 Hamburg (DE)**

(56) Entgegenhaltungen:
**EP-A1- 1 281 319          EP-A2- 0 689 767 WO-A1-2008/034224**

• **"Manusept basic - Hygienic and surgical hand disinfection", , 1. März 2010 (2010-03-01), Seiten 1-2, XP55029609, Gefunden im Internet: URL:http://www.bode-chemie.com/products/ha nds/product_information/manusept_basic_int . pdf [gefunden am 2012-06-12]**

Bemerkungen:
Die Akte enthält technische Angaben, die nach dem Eingang der Anmeldung eingereicht wurden und die nicht in dieser Patentschrift enthalten sind.

EP 2 654 420 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft eine antimikrobielle Zusammensetzung zur Händedesinfektion auf Basis von 80 - 95 Gew.-% Ethanol, 1-Propanol, 2-Propanol oder deren Mischungen, die hautpflegende Eigenschaften besitzt, die visuell durch das Aussehen der Zusammensetzung vermittelt werden.

Zur Händedesinfektion, insbesondere zur hygienischen Händedesinfektion, werden bevorzugt Alkohollösungen angewendet. Diese Präparate mit hoher Alkoholkonzentration zeigen ein breites Wirkungsspektrum gegenüber Bakterien, Pilzen und verschiedenen Viren. Da gerade bei medizinischem Personal, z. B. in Kliniken, eine häufige Desinfektion stattfindet, müssen die verwendeten Desinfektionsmittel neben einer ausreichenden Desinfektionswirkung auch eine gute Hautverträglichkeit aufweisen. Üblicherweise werden deshalb den Desinfektionsmitteln pflegende Hilfsstoffe zugesetzt.

EP-0930065-B1 beschreibt eine antimikrobielle Zusammensetzung mit Lotion-artiger Erscheinung auf Basis einer Mischung aus 40-80 Vol.-% C2- und/oder C3-Alkohol und Wasser, Acrylpolymer-Verdickern, antimikrobiellen kationischen Verbindungen sowie Weichmachern und silikonbasierten Wachsen und Ölen als Hautpflegekomponente. Bei höheren Alkoholkonzentrationen zeigen die Zusammensetzungen, insbesondere in Abwesenheit einer ausreichenden Menge Wassers, eine geringe Stabilität, sowie die Bildung kleinerer Partikel beim Verreiben der Zusammensetzung auf der Haut.

EP-1374847-A1 beschreibt antimikrobielle Hände-Lotionen, bei denen die flüssige Phase mit einem Verhältnis von Alkohol zu Wasser von 35 zu 65 Gew.-% bis 100 zu 0 Gew.-%, gebildet wird. Mit einem Gemisch aus mindestens zwei Emulgatoren wird eine Viskosität von mindestens 4.000 cps, vzw. 80.000 bis 500.000 cps eingestellt, wobei die Zusammensetzung keinen Verdicker enthält. Zur Verbesserung des Feuchtigkeitsgehalts der Haut werden Weichmacher zugefügt.

WO-2006/096239-A1 offenbart antimikrobielle Emulsionen mit 50-95 Gew.-% C2 bis C4-Alkohol, vzw. 62-75 Gew.-% C2 bis C4-Alkohol, vzw. Ethanol, vernetzten Acrylat-Verdickern und hautpflegenden Komponenten, wobei diese ausgewählt sein können aus einer Vielzahl an handelsüblichen Hautpflegekomponenten und silikonfreien Emulgatoren.

US 2004/0102429A1 beschreibt eine Zusammensetzung, die gegen Hautirritationen wirken soll und in Kombination mit alkoholbasierten Desinfektionsmitteln verwendet werden kann, wobei die Zusammensetzung mindestens zwei organische Zinksalze enthält. Es können darüber hinaus Gelbildner, Verdicker, Pflegestoffe, Silikonöle etc. eingesetzt werden.

EP1281319 beschreibt eine antimikrobielle Zusammensetzung enthaltend Ethanol, Propanol, Polyacrylat und Ölkomponenten, wie Heptamethylnonan, 2-Ethylhexyl-2-ethylhexanoat, Lanolinalkohol und Tetradecanol zur Handdesinfektion.

Die bekannten Zusammensetzungen enthalten als Hautpflegestoffe Silikonöle. Es sind klassische Tensidsysteme erforderlich, um die Formulierungen zu stabilisieren oder um eine Verdickung der Gesamtrezeptur zu erzielen. Das Hautgefühl bei der Anwendung steht bei diesen Zusammensetzungen nicht im Vordergrund.

In der Veröffentlichung mit dem Titel "Gele für die dermale Applikation" des Autors Rolf Daniels (Pharmazeutische Zeitung, Online-Ausgabe 43/ 2002, http://www.pharmazeutische-zeitung.de/index.php?id=titel_43_2002, zuletzt aufgesucht am 15.12.2010) werden emulsionsartige Systeme beschrieben, die aus einer hydrophilen kontinuierlichen Phase und einer lipophilen dispersen Phase bestehen. Dabei wird die disperse Phase nicht durch klassische Emulgatoren, sondern durch Makromoleküle stabilisiert. Die physikalische Stabilität dieser Hydrodispersionsgele wird durch eine feine Verteilung der Lipide und durch die Fließgrenze der Außenphase erreicht.

Weiterhin sind Desinfektionsmittel am Markt bekannt, die einen Alkoholgehalt von > 80 Gew.-% haben, aber nicht als Emulsion, sondern als klare, durchsichtige Gele vorliegen. Außerdem sind Emulsionen bekannt, die einen Alkoholgehalt von ca. 60 Gew.-% haben und weitere antimikrobielle Wirkstoffe enthalten und solche, die bei einem Alkoholgehalt von ca. 60 Gew.-% eine Emulgator-/Verdickerkombination und Pflegestoffe enthalten.

Es hat sich gezeigt, dass die Compliance, das heisst, die Beachtung von Vorschriften, der hygienischen Händedesinfektion nur ca. 50% beträgt. Dieses beruht häufig auf einem schlechten Hautgefühl bei der Anwendung alkoholischer Produkte. Es ist deshalb seit längerem das Ziel der Hersteller, Produkte bereitzustellen, die eine gute Hautverträglichkeit aufweisen. Hierfür werden unter anderem auch Gele und Lotionen auf den Markt gebracht. Der Nachteil der in diesen Zusammensetzungen eingesetzten Verdicker ist, dass das Produkt häufig als klebrig auf der Haut empfunden wird, was wieder zu einer geringeren Bereitschaft führt, das Produkt zu verwenden. Zudem tritt teilweise ein Abrieb auf der Haut auf, der auch als "Rubbelbildung" beschrieben wird. Sowohl die Klebrigkeit als auch die Rubbelbildung werden insbesondere als unangenehm empfunden, wenn mit Schutzhandschuhen gearbeitet wird, da hierdurch das Anlegen und Ausziehen erschwert wird. Zudem ist mit klebrigen Händen das Arbeiten mit Verbandmaterial oder die Vorbereitung oder Verabreichung von Arzneimitteln sehr erschwert. Sofern eine geringere Menge Verdicker eingesetzt wird, sind viele der Produkte, sobald sie einen hohen Alkoholgehalt besitzen, nicht ausreichend stabil. Für eine ausreichende Desinfektion ist der hohe Alkoholanteil jedoch notwendig.

Ausgehend von diesem Stand der Technik besteht somit das Problem, dass es keine stabilen Zusammensetzungen mit antimikrobieller Wirksamkeit gemäß DIN EN 1500 (Oktober 1999), bei gleichzeitig angenehmen Anwendungseigenschaften und guten Pflegeeigenschaften gibt, um die Compliance in der hygienischen Händedesinfektion zu verbessern.

Die Aufgabe der vorliegenden Erfindung besteht darin, ein hautpflegendes Desinfektionsmittel zu entwickeln, das eine hohe antimikrobielle Wirksamkeit gemäß DIN EN 1500, bei angenehmen Anwendungseigenschaften, wie z. B. keine Klebrigkeit des Produktes, keine Bildung von Abrieb auf der Haut, gute Verteilbarkeit und gutes Einziehvermögen und eine hohe Pflegewirkung, aufweist.

Die Aufgabe wird erfindungsgemäß gelöst durch eine antimikrobielle Zusammensetzung, enthaltend

a) 80 - 95 Gew.-% Ethanol, 1-Propanol, 2-Propanol oder Mischungen hiervon,
b) einen oder mehrere silikonhaltige Emulgatoren, wobei der Emulgator ausgewählt ist aus Copolymeren aus Dimethicon mit PEG und/oder PPG, mit gleichen oder unterschiedlichen Kettenlängen,
c) einen oder mehrere Verdicker auf Basis von quervernetzter Polyacrylsäure oder Acrylat/$C_{10-30}$ Alkylacrylat Crosspolymer,
d) mindestens zwei silikonfreie Ölkomponenten, ausgewählt aus ungesättigten oder gesättigten, linearen oder verzweigten aliphatischen Kohlenwasserstoffen,
Fettalkoholen, Fettsäureestern und deren Mischungen,
wobei die Ölkomponenten ausgewählt sind aus den Gruppen besitzend i) schnelles Spreitverhalten, ii) mittleres Spreitverhalten und iii) langsames Spreitverhalten, wobei mindestens zwei Ölkomponenten aus verschiedenen Gruppen i) - iii) ausgewählt sind,
e) Wasser dadurch gekennzeichnet, dass
die Zusammensetzung lösliche Ölkomponenten zu einem Anteil von mindestens 1,5 Gew.-% und unlösliche Ölkomponenten zu einem Anteil von höchstens 4,0 Gew.-% enthält, wobei der Gesamtanteil an löslichen und unlöslichen Ölkomponenten an der antimikrobiellen Zusammensetzung höchstens 6,0 Gew.-% beträgt und mindestens eine Ölkomponente eingesetzt wird, die in den Alkoholkomponenten unlöslich ist und der silikonhaltige Emulgator ausgewählt ist aus der Gruppe bestehend aus Cetyl PEG/PPG-10/1-Dimethicon, Bis-PEG/PPG-20/5 PEG/PPG-20/5-Dimethicon, und/oder Methoxy PEG/PPG-25/4-Dimethicon.
Diese Zusammensetzung vereint die Vorzüge einer pflegenden Lotion und die Anwendungseigenschaften eines Händedesinfektionsgeles. Die in EP 0 930 065B1 aufgezeigten Nachteile der Instabilität und der Partikelbildung beim Verreiben konnten überraschenderweise überwunden werden.
Weitere Ausführungsformen sind Gegenstand der Unteransprüche oder nachfolgend beschrieben.
Bevorzugt enthält die antimikrobielle Zusammensetzung mindestens eine weitere Komponente, ausgewählt aus
f) pH-Wert-Regulatoren,
g) Trübungsmitteln,
h) weiteren antimikrobiellen Wirkstoffen und
j) Co-Verdickern.

Die erfindungsgemäße antimikrobielle Zusammensetzung enthält bevorzugt a) 83 - 89 Gew.-%, besonders bevorzugt 85 - 87 Gew.-% Ethanol, 1-Propanol, 2-Propanol oder deren Mischungen. Der silikonhaltige Emulgator b) ist ein silikonhaltiger nichtionischer Emulgator ausgewählt aus der Gruppe bestehend aus Cetyl PEG/PPG-10/1-Dimethicon, Bis-PEG/PPG-20/5 PEG/PPG-20/5-Dimethicon, und/oder Methoxy PEG/PPG-25/4-Dimethicon.

Emulgatoren sind Verbindungen mit einem lipophilen (also öllöslichen) Anteil und einem chemisch daran fest gebundenen hydrophilen (wasserlöslichen) Anteil. Diese Verbindungen lagern sich an der Grenzfläche zwischen wässrigen und öligen Komponenten an und ermöglichen damit die Bildung einer Emulsion. Bei den verwendeten Emulgatoren handelt es sich um silikonhaltige Emulgatoren, deren lipophiler Teil aus einem Dimethicon (Polydimethylsiloxan) gebildet wird, während der hydrophile Teil aus wasserlöslichem Polyethylenglycol (PEG) und/oder Polypropylenglycol (PPG) gebildet wird.

Der Emulgator bzw. die Emulgatoren b) ist bzw. sind in einer Menge von 0,1 - 4,0 Gew.-%, bevorzugt 0,1 - 2,0 Gew.-%, besonders bevorzugt 0,1 - 0,9 Gew.-%, ganz besonders bevorzugt 0,2 - 0,7% in der Zusammensetzung enthalten. Der silikonbasierte nichtionische Emulgator ist ausgewählt aus Copolymeren aus Dimethicon mit PEG und/oder PPG, mit gleichen oder unterschiedlichen Kettenlängen, ausgewählt aus Cetyl PEG/PPG-10/1-Dimethicon, Bis-PEG/PPG-20/5 PEG/PPG-20/5-Dimethicon, Methoxy PEG/PPG-25/4-Dimethicon und/oder Dimethicon, weiter bevorzugt 0,1-0,9 Gew.-% Bis-PEG/PPG-20/5 PEG/PPG-20/5-Dimethicon, Methoxy PEG/PPG-25/4-Dimethicon. Die erfindungsgemäße antimikrobielle Zusammensetzung enthält bevorzugt keine anderen kationischen oder anionischen oder amphoteren Emulgatoren.

Die antimikrobielle Zusammensetzung enthält vorzugsweise 0,01 - 0,5 Gew.-%, besonders bevorzugt 0,1 - 0,3 Gew.-%, ganz besonders bevorzugt 0,15 - 0,27% mindestens eines Verdickers c) auf Basis von quervernetzter Polyacrylsäure oder deren Derivaten. Die Acrylsäurepolymere werden auch als Carbomere bezeichnet. Sie sind hochmolekulare Verbindungen und werden üblicherweise in der pharmazeutischen oder kosmetischen Industrie für die Verdickung, aber auch für Dispergierung und Emulsionsbildung topischer Zubereitungen eingesetzt. Bevorzugt handelt es sich bei dem Verdicker um Acrylat/$C_{10-30}$ Alkylacrylat Cross Polymer (INCI). Ein geeigneter Verdicker ist beispielsweise Carbopol® ETD 2020 NF oder Carbopol® Ultrez 10 NF von der Firma Lubrizol.

Die Verdickungsleistung bzw. die Viskosität der Zubereitung wird dabei bevorzugt eingestellt über die Menge des zugegebenen Co-Verdickers j), bevorzugt ausgewählt aus der Gruppe der Amine, vorzugsweise ausgewählt aus N,N,N',N'-Tetrakis-(hydroxyalkyl)-ethylendiamin, wobei die Hydroxyalkyl-Gruppe vorzugsweise 2-Hydroxypropyl ist. Der Co-Verdicker ist bevorzugt in einer Menge von 0 - 1,0 Gew.-%, besonders bevorzugt 0,01 - 1,0 Gew.-%, weiter bevorzugt 0,2 - 0,6 Gew.-%, ganz besonders bevorzugt 0,3 - 0,54 in der erfindungsgemäßen Zusammensetzung enthalten. Das Gewichtsverhältnis von Verdicker c) zu Co-Verdicker j) beträgt vorzugsweise 1:1,5 bis 1:2,5, bevorzugt 1:2.

Bevorzugt sind der oder die pH-Wert-Regulatoren f) ausgewählt aus Zitronensäure, Milchsäure, Weinsäure, Pyroglutaminsäure, Ascorbinsäure, Kaliumhydroxid und/oder Natriumhydroxid.

Die antimikrobielle Zusammensetzung enthält bevorzugt als Trübungsmittel g) unlösliche Substanzen, die zu einem weißlich-trüben Aussehen führen, wie beispielsweise pulverförmige Polyamid-Partikel (beispielsweise Orgasol® Caresse der Firma Arkema Inc.; INCI: Polyamide-5) oder wachsartige Partikel in Form von Mischungen aus Glycoldistearat, Cocoamidopropylbetain, Cocoamidmonoethanolamin, Natriumlaurethsulfat und/oder PEG-Laurylether (beispielsweise Euperlan® PK 771, PK 810, PK 900, PK 3000, PK 4000 oder PK 4500 von Cognis Corp.).

Die erfindungsgemäße antimikrobielle Zusammensetzung enthält mindestens zwei Ölkomponenten, bevorzugt enthält die erfindungsgemäße antimikrobielle Zusammensetzung mindestens 3 Ölkomponenten, weiter bevorzugt mindestens vier Ölkomponenten, weiter bevorzugt mindestens 5 Ölkomponenten, wobei die Ölkomponenten ausgewählt sind aus den Gruppen aufweisend:

> i) schnelles Spreitverhalten (hoch spreitende Öle),
> ii) mittleres Spreitverhalten (mittel spreitende Öle) und
> iii) langsames Spreitverhalten (niedrig spreitende Öle),

wobei zwei oder mehrere Ölkomponenten aus verschiedenen Gruppen i) bis iii) ausgewählt werden.

Unter Spreitverhalten wird die Fähigkeit einer Substanz verstanden, sich auf einem Festkörper auszubreiten. Ein Tropfen, der auf die Hautoberfläche auftrifft, benetzt die Oberfläche abhängig von seiner Oberflächenspannung und seiner Viskosität. Eine Substanz mit einer hohen Oberflächenspannung benetzt aufgrund der hieraus bedingten halbkugelförmigen Form nur eine geringe Oberfläche und weist somit ein schlechtes Spreitverhalten auf. Eine Substanz mit einer geringen Oberflächenspannung und einer niedrigen Viskosität benetzt eine große Hautoberfläche, da sie sich schneller auf der Oberfläche ausbreitet. Diese Substanz besitzt entsprechend ein schnelles Spreitverhalten, sie ist hoch spreitend. Es wird unterschieden zwischen niedrig spreitenden, mittel spreitenden und hoch spreitenden Ölen. Niedrig spreitende Öle besitzen ein Spreitverhalten von unter 450 mm$^2$/10 Minuten, mittel spreitende Öle haben ein Spreitverhalten von 450 - 1000 mm$^2$/ 10 Minuten und hoch spreitende Öle ein Spreitverhalten von oberhalb 1000 mm$^2$/10 Minuten. Der Spreitwert wird mit Hilfe eines Extensometers bestimmt. Dabei wird nach Voigt (R. Voigt, Pharmazeutische Technologie - Für Studium und Beruf, Wissenschaftliche Verlagsgesellschaft mbH Stuttgart, 9. Auflage, 2000, Seite 333 - 334) eine Stoffprobe bestimmten Volumens zentriert zwischen zwei Glasplatten gegeben und die obere Platte in definierten Intervallen mit Wägestücken beschwert. Die mit steigender Belastung resultierenden Ausbreitungsflächen stellen ein Charakteristikum für die Spreitbarkeit dar. Die Einteilung der oben genannten Spreitwerte kann beispielsweise nach Raab/Kindl erfolgen (W. Raab, U. Kindl, Pflegekosmetik - Ein Leitfaden, Wissenschaftliche Verlagsgesellschaft mbH Stuttgart, 4. Auflage, 2004, Seite 204 - 205).

Hoch spreitende Ölkomponenten sind beispielsweise Isopropylmyristat, Isopropylpalmitat, Cetarylethylhexanoat. Ölkomponenten mit mittlerem Spreitverhalten sind beispielsweise Ethylhexylpalmitat, Ethylhexylstearat, Heptamethylnonan, Capryl/Caprin-Triglycerid. Erfindungsgemäß geeignete Ölkomponenten mit einem langsamen Spreitverhalten sind beispielsweise Paraffinum subliquidum, Octyldodecanol, Isocetylpalmitat.

Die Spreiteigenschaften sind darüber hinaus abhängig von der Kettenlänge der verwendeten Ölkomponenten. Die langsam spreitenden Öle weisen zumeist eine längere Kohlenstoffkette und eine höhere Polarität auf als die schnell spreitenden Öle. Es hat sich gezeigt, dass durch Einarbeitung von Ölkomponenten mit unterschiedlichem Spreitverhalten das Hautgefühl und die Anwendungseigenschaften signifikant verbessert werden können. Durch die schnell spreitenden Öle entsteht zu Beginn der Anwendung ein positives Hautgefühl, während durch die Anwendung der mittel bzw. langsam spreitenden Öle auch nach der Anwendung ein angenehmes Pflegegefühl erhalten bleibt und hierdurch ein übergangsloses und verbessertes Einreibeverhalten erreicht wird. Werden die Ölkomponenten so gewählt, dass in Bezug auf die Kettenlänge bzw. das Spreitverhalten eine Abstufung über den Bereich von hoch bis niedrig spreitend umfasst wird, kann der Effekt eingestellt und verbessert werden. Es wird somit eine "Ölkaskade" bevorzugt eingesetzt, wobei mindestens zwei Ölkomponenten, bevorzugt zumindest 5 Ölkomponenten mit unterschiedlichem Spreitverhalten eingesetzt werden und hierdurch eine Ölkaskade ausbilden.

Damit ein angenehmes Pflegegefühl erreicht wird, sollten dafür vorzugsweise 50 % der Gesamtmenge der eingesetzten Ölkomponenten ein langsames Spreitverhalten aufweisen.

Die antimikrobielle Zusammensetzung enthält weiter mindestens 1,5 Gew.-% lösliche Ölkomponenten und gleichzeitig unlösliche Ölkomponenten zu höchstens 4,0 Gew.-%, wobei der Gesamtanteil an löslichen und unlöslichen Ölkomponenten an der antimikrobiellen Zusammensetzung höchstens 6,0 Gew.-% beträgt. Bevorzugt enthält die erfindungsgemäße antimikrobielle Zusammensetzung mindestens 1,5 Gew.-% Paraffinum subliquidum, Isopropylpalmitat oder Mischungen hiervon und Cetarylethylhexanoat, Myristylalkohol, Heptamethylnonan oder Mischungen hiervon zu höchstens 4,0 Gew.-%, wobei der Gesamtanteil an löslichen und unlöslichen Ölkomponenten an der antimikrobiellen Zusammensetzung höchstens 6,0 Gew.-% beträgt.

Es hat sich gezeigt, dass durch den Einsatz von Ölkomponenten, die in den Alkoholkomponenten unlöslich sind, eine Trübung der Lösung erreicht wird. Die Trübung der Lösung führt zu einem weißlichen bzw. weißen Produkt. Durch dieses Aussehen wird die pflegende Eigenschaft des Produktes optisch unterstützt. Da die Farbe Weiß in westlichen Kulturkreisen häufig als Symbol von Reinheit verstanden wird, werden weißen Produkten eher die Eigenschaften "reinigend" und "pflegend" zugeordnet. Die positiven hautpflegenden Eigenschaften werden somit farblich unterstrichen. Dies sollte zu einer Verbesserung der Compliance der Händedesinfektion führen. Die Verwendung von Silikonölen, wie z. B. Polydimethylsiloxan (Dimethicon) oder Decamethylcyclopentasiloxan (Cyclomethicon), unterstützt die optische Erscheinung der erfindungsgemäßen Emulsionen nicht, da die genannten Silikonöle klardurchsichtige ölige Flüssigkeiten sind, die in Alkoholen löslich sind.

Überraschenderweise gelang es, eine stabile, trübe Zusammensetzung zu erhalten, obwohl zu erwarten war, dass bei einem Alkoholgehalt von 80 % und mehr die Ölkomponenten in klarer Lösung vorliegen würden.

Die erfindungsgemäße antimikrobielle Zusammensetzung liegt bevorzugt als Emulsion, Lotion, Gel oder Hydrodispersionsgel, besonders bevorzugt als Emulsion oder Hydrodispersionsgel vor.

In der erfindungsgemäßen antimikrobiellen Zusammensetzung können zudem neben den einwertigen $C_2$-/$C_3$-Alkoholen weitere antimikrobielle

Wirkstoffe/Remanenzwirkstoffe h) enthalten sein, wobei diese bevorzugt in einer Menge von 0 - 5,0 Gew.-% enthalten sind. Geeignete antimikrobielle Wirkstoffe sind vorzugsweise ausgewählt aus der Gruppe der anionischen und nichtionischen Wirkstoffe, bevorzugt aus Diolen oder Phenoxyethanol.

In einer bevorzugten Ausführungsform besteht die erfindungsgemäße antimikrobielle Zusammensetzung aus

a) 80 - 95 Gew.-% Ethanol, 1-Propanol, 2-Propanol oder Mischungen hiervon

b) 0,1 - 4,0 Gew.-% eines oder mehrerer silikonhaltiger Emulgatoren, ausgewählt ist aus Copolymeren aus Dimethicon mit PEG und/oder PPG, mit gleichen oder unterschiedlichen Kettenlängen, wie vorstehend beschrieben,

c) 0,01 - 0,5 Gew.-% eines oder mehrerer Verdicker auf Basis von quervernetzter Polyacrylsäure und Acrylat/$C_{10-30}$Alkylacrylat Crosspolymer,

d) 1,5 - 6,0 Gew.-% mindestens zweier silikonfreier Ölkomponenten, ausgewählt aus hautpflegenden Kohlenwasserstoffen, Fettalkoholen, Fettsäureestern und deren Mischungen,

wobei die Ölkomponenten ausgewählt sind aus den Gruppen besitzend i) schnelles Spreitverhalten, ii) mittleres Spreitverhalten und iii) langsames Spreitverhalten, wobei mindestens zwei Ölkomponenten aus verschiedenen Gruppen i) - iii) ausgewählt sind,

e) 4 - 18 Gew.-% Wasser,

j) 0,01-1,0 Gew.-%, vorzugsweise 0,2-0,6 Gew.-% Co-Verdicker, ausgewählt aus N,N,N',N'-Tetrakis-(hydroxyalkyl)-ethylendiamin, wobei die Hydroxyalkylgruppe vorzugsweise 2-Hydroxypropyl ist, wobei vorzugsweise das Gewichtsverhältnis c) : j) 1:1,5 bis 1:2,5 und bevorzugt 1:2 beträgt.

Die erfindungsgemäße antimikrobielle Zusammensetzung weist bevorzugt eine dynamische Viskosität von 1 bis 12 Pa*s, vzw. 1 bis 5 Pa*s auf, gemessen bei 20°C (+/- 0,5°C) mit einem Brookfield Rotationsviskosimeter Model DV-II+, Spindel Größen 2, 3, 4 oder 5 bei 10 rpm. Die Viskosität kann z. B. durch Einsatz des Verdickers entsprechend eingestellt werden. Die Viskosität wird bevorzugt so eingestellt, dass sich die erfindungsgemäße antimikrobielle Zusammensetzung gut auf der zu desinfizierenden Oberfläche auftragen lässt und ein angenehmes Einreibeverhalten aufweist.

Die als weiße Farbe wahrgenommene Trübung ergibt sich bevorzugt aus einem Trübungswert von mindestens 200 NTU, vorzugsweise mindestens 500 NTU, besonders bevorzugt mindestens 800 NTU. Die Bestimmung der Trübung der erfindungsgemäßen antimikrobiellen Zusammensetzungen erfolgte nach einer optischen Methode, die im Europäischen Arzneibuch (2.2.1 Ph. Eur., 6. Ausgabe, Grundwerk 2008) beschrieben wird. Dabei handelt es sich um eine Prüfung gegenüber einer Reihe von Referenzproben mit definiertem Trübungsgrad mittels einer 90°-Streulichtmessung. Die Referenzproben bestehen aus einer Dispersion aus Formazin (einem Reaktionsprodukt aus Hexamethylentetramin und Hydraziniumsulfat) in Aqua purificata. Die Einheit NTU (Nephelometric Turbidity Unit) entspricht dabei der deutschen Einheit TE/F (Trübungseinheit Formazin). Es handelt sich bei der Trübungsbestimmung um ein relatives Messverfahren, da die Trübung keine physikalische Größe darstellt. Das erfindungsgemäße Händedesinfektionsmittel weist einen pH-Wert im Bereich von 6-9, bevorzugt von 7-9, besonders bevorzugt von 8-9 auf. Sofern nicht anders beschrieben, beziehen sich die Gew.-%-Angaben jeweils auf das Gewicht der Gesamtzusammensetzung.

Die erfindungsgemäße antimikrobielle Zusammensetzung wird als Desinfektionsmittel, insbesondere zur Desinfektion von belebten Oberflächen, insbesondere zur Händedesinfektion, besonders bevorzugt zur hygienischen und chirurgischen Händedesinfektion verwendet. Zur Überprüfung der Wirksamkeit wurde die erfindungsgemäße antimikrobielle Zusammensetzung einer Prüfung gemäß DIN EN 1500 (Oktober 1997) unterzogen. DIN EN 1500 legt ein Prüfverfahren fest, das die praktische Anwendung eines Produktes für die hygienische Händedesinfektion simuliert. Durch die Reduktion der transienten Hautflora auf den künstlich kontaminierten Händen freiwilliger Probanden wird dabei festgelegt, ob das Prüfprodukt die Anforderungen erfüllt. Die hygienische Händedesinfektion ist dabei definiert als "Behandlung der Hände nach einer Kontamination durch Einreiben (ohne Zugabe von Wasser) mit einem bakteriziden Produkt gegen transiente Mikroorganismen, um deren Übertragung zu verhindern; hierbei wird die residente Hautflora nicht berücksichtigt." Die Reduktion wird dabei mit Hilfe des sogenannten Reduktionsfaktors (RF) ausgedrückt. Der RF ist die Differenz aus dem dekadischen Logarithmus der Anzahl koloniebildender Einheiten (KBE) vor und nach der Behandlung:

$$\text{lg RF} = \text{lg Vorwert (KBE)} - \text{lg Nachwert (KBE)}$$

Die erreichte mittlere Verringerung der Prüfkeime darf dabei nicht signifikant geringer sein als die durch eine Referenz-Händedesinfektion mit 60 Vol.-% 2-Propanol erreichte Verringerung.

Durchführung:

Die Hände von mindestens 12 Probanden werden für eine Minute mit Kaliseife gewaschen und anschließend mit Papierhandtüchern abgetrocknet. Die Hände werden danach bis zur Mittelhand für 5 Sekunden in die Kontaminationsflüssigkeit mit E. coli Bakterien (Stamm K 12, NCTC 10538) gehalten. Danach werden die Hände für 3 Minuten an der Luft getrocknet und unmittelbar danach werden die Fingerkuppen inklusive Daumen in einer Petrischale je Hand mit 10 mL eines Nährmediums (Trypton-Soja-Lösung, TSL) für eine Minute ausgeknetet (Vorwert). Unmittelbar im Anschluss daran und ohne weitere Kontamination wird die Händedesinfektion durchgeführt, entweder mit dem Referenzalkohol oder mit dem Prüfprodukt (Referenzalkohol: 2 mal 3 mL für je 30 Sekunden gemäß Standard-Einreibeverfahren DIN EN 1500 Anhang A verreiben, Prüfprodukt: nach Herstellerangaben, hier: 3 mL für 30 Sekunden). Nach der Desinfektion werden die Finger für 5 Sekunden mit Leitungswasser abgespült und erneut in einer Petrischale je Hand mit 10 mL eines Nährmediums für eine Minute ausgeknetet (Nachwert).

Von den Nährmedien werden jeweils 1,0 mL und 0,1 mL der unverdünnten Flüssigkeit der linken und der rechten Hand auf insgesamt 4 CSA-Platten ausgespatelt. Innerhalb von 30 Minuten nach der Probenahme werden die CSA-Platten für 24 h bei 36 °C bebrütet und die Kolonien ausgezählt.

Die Summe der KBE zweier aufeinanderfolgender Verdünnungen werden durch den Verdünnungsfaktor geteilt, der erhaltene Wert entspricht dann KBE/mL unverdünnter Sammelflüssigkeit:

$$[\text{n KBE (von 1,0 mL)} + \text{n KBE (von 0,1 mL)}] / 1,1 * 10\text{-}1$$

$$= \text{Anzahl KBE/mL unverdünnter Sammelflüssigkeit.}$$

Alle Keimzahlen je Milliliter Sammelflüssigkeit werden dekadisch logarithmiert. (Aus rechnerischen Gründen müssen die Werte von "0" ($\text{lg } 0 = \infty$) gleich "1" ($\text{lg } 1 = 0$) gesetzt werden.

Es hat sich gezeigt, dass die Verwendung der erfindungsgemäßen antimikrobiellen Zusammensetzung zu einer log 3 Reduktion gemäß DIN EN 1500 führt.

Ein weiteres Standardverfahren für den Nachweis einer antimikrobiellen Wirksamkeit ist in DIN EN 13727 (Entwurf Dezember 2009) verankert. DIN EN 13727 beschreibt ein Prüfverfahren und die Mindestanforderungen an die bakterizide Wirkung von chemischen Desinfektionsmitteln und antiseptischen Produkten zur Feststellung, ob ein Antiseptikum im Rahmen des im Anwendungsbereich beschriebenen Arbeitsgebiets und Feldes eine bakterizide Wirkung aufweist oder nicht. Die Produkte können nur in einer Konzentration von 80 % oder darunter geprüft werden, da durch Zugabe der Prüfkeime und der Belastungssubstanz immer eine gewisse Verdünnung bewirkt wird. Die Prüfkeime für eine hygienische oder chirurgische Händedesinfektion sind *Pseudomonas aeruginosa* (ATCC 15442), *Staphylococcus aureus* (ATCC 6538), *Enterococcus hirae* (ATCC 10541) und *Escherichia coli* K12 (NCTC 10538).

Die Prüftemperatur beträgt 20 °C, die Einwirkzeit für eine hygienische Händedesinfektion sind 60 Sekunden, für eine chirurgische Händedesinfektion 5 Minuten. Darüber hinaus werden Einwirkzeiten nach Herstellerangaben geprüft. Produktprüflösungen sind in mindestens drei unterschiedlichen Konzentrationen in Wasser standardisierter Härte herzustellen, wobei eine Konzentration im wirksamen Bereich und eine Konzentration im nicht wirksamen Bereich liegen muss. Das Produkt im Lieferzustand darf als eine der Produktprüflösungen verwendet werden; in diesem Fall beträgt die höchste geprüfte Konzentration 80 Gew.-%. Die Anzahl der Zellen in der Prüfsuspension wird mit dem Verdünnungsmittel auf 1,5x108 KBE je ml bis 5,0x108 KBE je ml eingestellt. Die Belastungssubstanz muss nach den für das Produkt festgelegten Anwendungsbedingungen ausgewählt werden. Sie muss steril sein und mit einer zehnfach höheren Konzentration als für die Prüfung benötigt wird, hergestellt werden. Bei geringer Belastung wird Rinderserumalbuminlösung in niedriger Konzentration verwendet. Bei hoher Belastung wird ein Gemisch von Rinderserumalbuminlösung in hoher Konzentration mit Schaf-Erythrozyten verwendet. Zur Kontrolle wird statt einer Prüfsuspension eine Validierungssuspension verwendet, bei der die Prüfsuspension mit dem Verdünnungsmittel so verdünnt wird, dass sich 3,0x102 KBE je ml bis 1,6x103 KBE je ml ergeben.

Es werden 1,0 ml Belastungssubstanz und 1,0 ml Prüfsuspension in ein Reagenzglas pipettiert. Die Stoppuhr wird sofort in Gang gesetzt, es wird durchmischt und das Reagenzglas für 2 min +/- 10 s in ein auf 20 °C eingestelltes Wasserbad gestellt. Nach Ablauf dieser Zeit werden 8,0 ml einer der Produktprüflösungen zugegeben. Die Stoppuhr wird bei Beginn der Zugabe wieder in Gang gesetzt, es wird durchmischt und das Reagenzglas für die gewählte Einwirkzeit t in ein auf 20 °C eingestelltes Wasserbad gestellt.

Unmittelbar vor dem Ende von t wird die Lösung erneut durchmischt. Nach Ablauf von t wird eine 1,0 ml-Probe des Prüfgemischs in ein Röhrchen mit 8,0 ml Neutralisationsmedium und 1,0 ml Wasser überführt. Es wird durchmischt und in ein auf 20°C eingestelltes Wasserbad gestellt. Nach einer Neutralisationszeit von (10 +/- 1) s (Sekunden) wird durchmischt und sofort eine Probe von 1,0 ml des neutralisierten Prüfgemischs (enthaltend Neutralisationsmedium, Produktprüflösung, Belastungssubstanz und Prüfsuspension) als Doppelbestimmung entnommen und im Gussplatten- oder Oberflächenverfahren beimpft.

Zusätzlich werden 0,5 ml dieses Gemischs in ein Röhrchen überführt, das 4,5 ml des Neutralisationsmediums enthält, um das neutralisierte Prüfgemisch in einer Verdünnung von 10-1 zu erhalten. Von jedem Verdünnungsröhrchen werden Proben von 1,0 ml als Doppelbestimmung entnommen und im Oberflächenverfahren beimpft.

Bei der Verwendung des Oberflächenverfahrens wird jede 1,0-ml-Probe, die in Anteile etwa gleicher Größe aufgeteilt ist, auf eine geeignete Anzahl (mindestens zwei) von auf der Oberfläche getrockneten Platten mit TSA ausgestrichen.

Die Einwirkzeiten betrugen 15, 30 und 60 Sekunden bei einer Produktkonzentration von 40 Gew.-%. Die Platten werden 24 h bebrütet und zur Bestimmung der Anzahl der koloniebildenden Einheiten (KBE) ausgezählt. Bei Prüfung unter simulierten Bedingungen niedriger Belastung (0,3 g/l Rinderserumalbuminlösung) oder unter Bedingungen hoher Belastung (3 g/l Rinderserumalbuminlösung und 3 ml/l gewaschene Schaf-Erythrozyten) muss ein Produkt zur Händedesinfektion eine Verminderung um mindestens fünf dekadisch-logarithmische Stufen (log 5) nachweisen.

Es hat sich gezeigt, dass die Verwendung der erfindungsgemäßen antimikrobiellen Zusammensetzung zu einer log 5 Reduktion gemäß DIN EN 13727 führt.

Weiter erfindungswesentlich ist das Verfahren zur Herstellung der erfindungsgemäßen antimikrobiellen Zusammensetzungen. Dem Fachmann ist bekannt, dass Fette und Öle in Alkoholen zum Teil eine gute Löslichkeit aufweisen können und dass Öl-in-Wasser-Emulsionen, bei denen in der Wasserphase ein hoher Alkoholanteil vorliegt, schwierig zu stabilisieren sind. Die Instabilität zeigt sich beispielsweise durch eine vollständige Lösung der Ölphase in der alkoholisch-wässrigen Phase. Vor diesem Hintergrund stellt die Aufgabe der Herstellung einer lagerstabilen, hydroalkoholischen Emulsion, bei der die alkoholisch-wässrige Phase die Eigenschaften eines Geles aufweist, eine besondere Herausforderung dar.

Gegenstand der Erfindung ist daher weiter ein Verfahren zur Herstellung der vorbeschriebenen erfindungsgemäßen antimikrobiellen Zusammensetzung. Das Verfahren umfasst bevorzugt die folgenden Schritte, die nacheinander durchgeführt werden:

1. Zu einer Mischung M1 aus

a1) 8 - 15 Gew.-% Ethanol, 1-Propanol, 2-Propanol oder Mischungen hiervon,
c) 0,1 - 0,3 Gew.-% mindestens eines Verdickers und
e1) 4 - 18 Gew.-% Wasser
wird eine Mischung M2, bestehend aus
a2) 70 - 80 Gew.-% Ethanol, 1-Propanol, 2-Propanol oder Mischungen hiervon,
j) Co-Verdickern und
d1) mindestens einer in der Mischung aus a2) und j) löslichen Ölkomponente, zugegeben und zur Gelbildung verrührt.

2. In einer Mischung aus

e2) 0 - 14 Gew.-% Wasser, wobei sich e1) und e2) zu 4 - 18 Gew.-% Wasser ergänzen, und
b) 0,1 - 4,0 Gew.-% eines silikonhaltigen Emulgators
wird unter Rühren
d2) mindestens eine unlösliche Ölkomponente emulgiert, wobei die Ölkomponenten d1) und d2) ausgewählt sind aus ungesättigten oder gesättigten, linearen oder verzweigten aliphatischen Kohlenwasserstoffen, Fettalkoholen, Fettsäureestern und deren Mischungen.

3. Die in Schritt 2 gebildete Emulsion (Mischung M3) wird in die Gelphase eingebracht und unter langsamem Rühren zu einer homogenen Zubereitung verarbeitet.

Durch das Verfahren wird eine stabile Emulsion erhalten, die trotz des hohen Anteils an Alkohol eine ausreichende Langzeitstabilität aufweist. Bei dem zuvor beschriebenen Verfahren wird somit zunächst ein Gel erzeugt und anschließend erfolgt die Einarbeitung der Ölphase zur Herstellung einer hydroalkoholischen Emulsion. Erfindungsgemäß ist das erfindungsgemäße antimikrobielle Mittel über eine alternative Verfahrensweise herstellbar. Bei diesem alternativen Verfahren werden die folgenden Schritte durchlaufen:

1. Zu einer Mischung M4 aus

a1) 8 - 15 Gew.-% Ethanol, 1-Propanol, 2-Propanol oder Mischungen hiervon,
c) 0,1 - 0,3 Gew.-% mindestens eines Verdickers
e) 4 - 18 Gew.-% Wasser
wird eine Mischung M5 aus
b) 0,1 -4,0 Gew.-% eines silikonhaltigen Emulgators und
d1) 0,5 - 6,0 Gew.-% einer Mischung aus mindestens 3 Ölkomponenten zugegeben.

2. Die Mischung wird so lange gerührt, bis sich eine Emulsion bildet.

3. Die gebildete Emulsion wird mit einer Mischung M6 aus

a2) 70 - 80 Gew.-% Ethanol, 1-Propanol, 2-Propanol oder Mischungen hiervon,
j) Co-Verdickern und
d2) mindestens einer in der Mischung aus a2) und j) löslichen Ölkomponente versetzt, wobei die Ölkomponenten d1) und d2) ausgewählt sind aus ungesättigten oder gesättigten, linearen oder verzweigten aliphatischen Kohlenwasserstoffen, Fettalkoholen, Fettsäureestern und deren Mischungen.

4. Nach Zugabe der Mischung M6 wird die Zusammensetzung weiter unter Rühren emulgiert und bei niedriger Rührgeschwindigkeit weiter gerührt.

Bei dieser Verfahrensweise wird somit zunächst eine Emulsion hergestellt, in die anschließend die löslichen Ölkomponenten und das Neutralisierungsmittel eingearbeitet werden. Die Verdickung der Zubereitung erfolgt somit am Ende des Verfahrens zur Herstellung einer hydroalkoholischen Emulsion.
Bei dem erfindungsgemäßen Verfahren werden die Verdicker, Ölkomponenten, silikonhaltigen Emulgatoren und Neutralisationsmittel eingesetzt, die auch für die erfindungsgemäße antimikrobielle Zusammensetzung verwendet werden. Diese werden auch in den bevorzugten Mengen wie bei der antimikrobiellen Zusammensetzung eingesetzt.
Die Erfindung wird anhand des nachfolgenden Beispiels erläutert.

Beispiel 1: Herstellung einer erfindungsgemäßen antimikrobiellen Zusammensetzung.

| Rohstoff | INCI- Bezeichnung | Menge [Gew.-%] |
|---|---|---|
| Aqua purificata | Gereinigtes Wasser | 10,16 |

(fortgesetzt)

| Rohstoff | INCI- Bezeichnung | Menge [Gew.-%] |
|---|---|---|
| Ethanol 99%, 2-Butanon vergällt | Ethanol | 6 |
| Tegosoft® Liquid | Cetarylethylhexanoat | 0,25 |
| Tegosoft® P | Isopropylpalmitat | 0,25 |
| Arlamol® HD | Heptamethylnonan | 0,25 |
| Paraffinum subliquidum | Dickflüssiges Paraffin | 1,8 |
| Abil® Care XL 80 | Bis-PEG/PPG-20/5 PEG/PPG-20/5 Dimethicon; Methoxy PEG/PPG-25/4 Dimethicon; Capryl-/Caprin-Triglycerid | 0,5 |
| Carbopol® ETD 2020 NF | Acrylat C10-30 Alkylacrylat CrossPolymer | 0,2 |
| Ethanol 99%, 2-Butanon vergällt | Ethanol | 80 |
| Lorol® C$_{14}$/Nacol® 14-95 | Myristylalkohol oder Tetradecanol | 0,19 |
| Neutrol® TE | Tetrahydroxypropylethylendiamin | 0,4 |
|  |  | 100,0 |

In einem 2-Liter Becherglas wurden 60,0 g Ethanol mit dem Wasser vorgelegt. In einem zweiten Becherglas wurde der Verdicker eingewogen und dann die Öle Cetarylethylhexanoat, Isopropylpalmitat, Heptamethylnonan und Paraffinum subliquidum sowie der silikonhaltige Emulgator hinzugegeben. Die Komponenten wurden miteinander verrührt. Diese Ölphase wurde unter Rühren mit einem Ankerrührer bei einer Rührergeschwindigkeit von 250- 800 rpm in die ethanolische Wasserphase emulgiert. Zur Bildung einer Emulsion wurde ca. weitere 30 Minuten bei einer Rührergeschwindigkeit von 250- 800 rpm gerührt.

Der Co-Verdicker Neutrol® TE und die Ölkomponente Lorol® C14 wurden in dem restlichen Ethanol gelöst und unter weiterem Rühren bei einer Rührergeschwindigkeit von 400- 800 rpm zur Emulsion gegeben. Die Zubereitung wurde solange weitergerührt, bis eine homogene agglomeratfreie Emulsion entstanden war.

Die erhaltene antimikrobielle Zusammensetzung wies einen pH-Wert im Bereich von 8-9 auf.

Die Viskosität nach 48 Stunden Lagerung bei Raumtemperatur betrug 2600 - 3800 mPa*s bei 20°C. Die Viskosität wird mit einem Brookfield Rotationsviskosimeter (Spindel 2) gemessen. Zur Bestimmung wurde die Zubereitung in ein 600 ml Becherglas gegeben, im Wasserbad auf 20 °C (+/- 0,5 °C) temperiert und 20 Messungen im Abstand von jeweils 15 Sekunden bei 10 rpm erfasst. Die Messwerte wurden abschließend gemittelt.

Beispiel 2: Herstellung einer weiteren erfindungsgemäßen Zusammensetzung

| Rohstoff | INCI- Bezeichnung | Menge [Gew.-%] |
|---|---|---|
| Aqua purificata | Gereinigtes Wasser | 10,542 |
| Ethanol 99%, 2-Butanon vergällt | Ethanol | 5,858 |
| Arlamol® HD | Heptamethylnonan | 0,500 |
| Paraffinum subliquidum | Dickflüssiges Paraffin | 1,500 |

(fortgesetzt)

| Rohstoff | INCI- Bezeichnung | Menge [Gew.-%] |
|---|---|---|
| Abil® Care XL 80 | Bis-PEG/PPG-20/5 PEG/PPG-20/5 Dimethicon; Methoxy PEG/PPG-25/4 Dimethicon; Capryl/Caprin Triglycerid | 1,000 |
| Carbopol® ETD 2020 NF | Acrylat C10-30 Alkylacrylat CrossPolymer | 0,200 |
| Ethanol 99%, 2-Butanon vergällt | Ethanol | 80,000 |
| Neutrol® TE | Tetrahydroxypropylethylendiamin | 0,400 |
| | | 100,000 |

Die antimikrobielle Zusammensetzung gemäß Beispiel 2 wurde analog Beispiel 1 hergestellt.

Beispiel 2 ist eine Rezeptur mit weniger Ölkomponenten und weniger Gesamt-Öl-Gehalt als Beispiel 1.

Beispiel 3: Herstellung einer weiteren erfindungsgemäßen Zusammensetzung

| Rohstoff | INCI- Bezeichnung | Menge [Gew.-%] |
|---|---|---|
| Aqua purificata | Gereinigtes Wasser | 7,542 |
| Ethanol 99%, 2-Butanon vergällt | Ethanol | 5,858 |
| Tegosoft® P | Isopropylpalmitat | 0,500 |
| Arlamol® HD | Heptamethylnonan | 0,500 |
| Paraffinum subliquidum | Dickflüssiges Paraffin | 3,000 |
| Abil® Care XL 80 | Bis-PEG/PPG-20/5 PEG/PPG-20/5 Dimethicon; Methoxy PEG/PPG-25/4 Dimethicon; Capryl-/Caprin-Triglycerid | 1,000 |
| Carbopol® ETD 2020 NF | Acrylat C10-30 Alkylacrylat CrossPolymer | 0,200 |
| Ethanol 99%, 2-Butanon vergällt | Ethanol | 80,000 |
| Lorol® $C_{14}$/Nacol 14-95 | Myristylalkohol oder Tetradecanol | 1,000 |
| Neutrol® TE | Tetrahydroxypropylethylendiamin | 0,400 |
| | | 100,000 |

Die antimikrobielle Zusammensetzung gemäß Beispiel 3 wurde analog Beispiel 1 hergestellt.

**Patentansprüche**

1.  Antimikrobielle Zusammensetzung, enthaltend

   a) 80 - 95 Gew.-% Ethanol, 1-Propanol, 2-Propanol oder Mischungen hiervon,
   b) einen oder mehrere silikonhaltige Emulgatoren, wobei der Emulgator ausgewählt ist aus Copolymeren aus Dimethicon mit PEG und/oder PPG, mit gleichen oder unterschiedlichen Kettenlängen,

c) einen oder mehrere Verdicker auf Basis von quervernetzter Polyacrylsäure oder Acrylat/C$_{10-30}$ Alkylacrylat Crosspolymer,

d) mindestens zwei Ölkomponenten, ausgewählt aus ungesättigten oder gesättigten, linearen oder verzweigten aliphatischen Kohlenwasserstoffen, Fettalkoholen, Fettsäureestern und deren Mischungen, wobei die Ölkomponenten ausgewählt sind aus den Gruppen besitzend i) schnelles Spreitverhalten, ii) mittleres Spreitverhalten und iii) langsames Spreitverhalten, wobei mindestens zwei Ölkomponenten aus verschiedenen Gruppen i) - iii) ausgewählt sind,

e) Wasser, **dadurch gekennzeichnet, dass**

die Zusammensetzung lösliche Ölkomponenten zu einem Anteil von mindestens 1,5 Gew.-% und unlösliche Ölkomponenten zu einem Anteil von höchstens 4,0 Gew.-% enthält, wobei der Gesamtanteil an löslichen und unlöslichen Ölkomponenten an der antimikrobiellen Zusammensetzung höchstens 6,0 Gew.-% beträgt, mindestens eine Ölkomponente eingesetzt wird, die in den Alkoholkomponenten unlöslich ist und der silikonhaltige Emulgator ausgewählt ist aus der Gruppe bestehend aus Cetyl PEG/PPG-10/1-Dimethicon, Bis-PEG/PPG-20/5 PEG/PPG-20/5-Dimethicon, und/oder Methoxy PEG/PPG-25/4-Dimethicon, weiter bevorzugt 0,1-0,9 Gew.-% Bis-PEG/PPG-20/5 PEG/PPG-20/5-Dimethicon, Methoxy PEG/PPG-25/4-Dimethicon.

2.  Antimikrobielle Zusammensetzung gemäß Anspruch 1, enthaltend mindestens eine weitere Komponente, ausgewählt aus

    f) pH-Wert-Regulatoren,
    g) Trübungsmitteln,
    h) weiteren antimikrobiellen Wirkstoffen,
    j) Co-Verdickern.

3.  Antimikrobielle Zusammensetzung gemäß Anspruch 2, **dadurch gekennzeichnet, dass** ein oder mehrere Co-Verdicker j) verwendet werden, die aus der Gruppe der Amine ausgewählt werden.

4.  Antimikrobielle Zusammensetzung gemäß einem der vorangehenden Ansprüche, enthaltend a) 83-89 Gew.-% Ethanol, 1-Propanol, 2-Propanol oder Mischungen hiervon, vorzugsweise 85-87 Gew.-% Ethanol, 1-Propanol, 2-Propanol oder Mischungen hiervon.

5.  Antimikrobielle Zusammensetzung gemäß einem der vorangehenden Ansprüche, enthaltend b) 0,1-4,0 Gew.-%, bevorzugt 0,1 - 2,0 Gew.-%, besonders bevorzugt 0,1 - 0,9 Gew.-% einen oder mehrere silikonhaltige Emulgatoren, ausgewählt aus Cetyl PEG/PPG-10/1-Dimethicon, Bis-PEG/PPG-20/5 PEG/PPG-20/5-Dimethicon, und/oder Methoxy PEG/PPG-25/4-Dimethicon, weiter bevorzugt 0,1-0,9 Gew.-% Bis-PEG/PPG-20/5 PEG/PPG-20/5-Dimethicon, Methoxy PEG/PPG-25/4-Dimethicon, wobei vorzugsweise keine anderen kationischen oder anionischen oder amphoteren Emulgatoren enthalten sind.

6.  Antimikrobielle Zusammensetzung gemäß einem der vorangehenden Ansprüche, enthaltend c) 0,01-0,5 Gew.-% mindestens eines Verdickers auf Basis von quervernetzter Polyacrylsäure oder Acrylat/C$_{10-30}$ Alkylacrylat Crosspolymer, vorzugsweise 0,1-0,3 Gew.-% mindestens eines Verdickers auf Basis von quervernetzter Polyacrylsäure oder Acrylat/C$_{10-30}$ Alkylacrylat Crosspolymer, und gegebenenfalls j) 0,01-1,0 Gew.-%, vorzugsweise 0,2-0,6 Gew.-% Co-Verdicker, vorzugsweise ausgewählt aus N,N,N',N'-Tetrakis-(hydroxyalkyl)-ethylendiamin, wobei die Hydroxyalkylgruppe vorzugsweise 2-Hydroxypropyl ist, wobei vorzugsweise das Gewichtsverhältnis c) : j) 1:1,5 bis 1:2,5 und bevorzugt 1:2 beträgt.

7.  Antimikrobielle Zusammensetzung gemäß einem der vorangehenden Ansprüche, enthaltend d) mindestens drei Ölkomponenten, vorzugsweise mindestens vier Ölkomponenten, weiter bevorzugt mindestens fünf Ölkomponenten.

8.  Antimikrobielle Zusammensetzung gemäß einem der vorangehenden Ansprüche, enthaltend mindestens 1,5 Gew.-% Paraffinum subliquidum, Isopropylpalmitat oder Mischungen hiervon und höchstens 4,0 Gew.-%Cetarylethylhexanoat, Myristylalkohol, Heptamethylnonan oder Mischungen hiervon, wobei der Anteil an löslichen und unlöslichen Ölkomponenten an der antimikrobiellen Zusammensetzung höchstens 6,0 Gew.-% beträgt.

9.  Antimikrobielle Zusammensetzung gemäß einem der vorangehenden Ansprüche, enthaltend

    f) 0-1,0 Gew.-% pH-Wert-Regulatoren, ausgewählt aus Zitronensäure, Milchsäure, Weinsäure, Pyroglutaminsäure, Ascorbinsäure, Kaliumhydroxid und/oder Natriumhydroxid,

g) 0- 5 Gew.-% Trübungsmittel, ausgewählt aus unlöslichen Substanzen, die zu einem weißlich-trüben Aussehen führen, vorzugsweise ausgewählt aus pulverförmigen Polyamid-Partikeln oder wachsartigen Partikeln in Form von Mischungen aus Glycoldistearat, Cocoamidopropylbetain, Cocoamidmonoethanolamin, Natriumlaurethsulfat und/oder PEG-Laurylether.

10. Antimikrobielle Zusammensetzung gemäß einem der vorangehenden Ansprüche, enthaltend h) 0-5,0 Gew.-% mindestens eines weiteren antimikrobiellen Wirkstoffes, ausgewählt aus weiteren antimikrobiellen Wirkstoffen/Remanenzwirkstoffen, vorzugsweise ausgewählt aus der Gruppe der anionischen und nichtionischen Wirkstoffe, bevorzugt Diolen oder Phenoxyethanol.

11. Antimikrobielle Zusammensetzung gemäß einem der vorangehenden Ansprüche, **dadurch gekennzeichnet, dass** die antimikrobielle Zusammensetzung als Emulsion, Lotion, Gel oder als Hydrodispersionsgel vorliegt, bevorzugt als Emulsion oder Hydrodispersionsgel.

12. Antimikrobielle Zusammensetzung gemäß einem der vorangehenden Ansprüche, bestehend aus

a) 80 - 95 Gew.-% Ethanol, 1-Propanol, 2-Propanol oder Mischungen hiervon,
b) 0,1 - 4,0 Gew.-% eines oder mehrerer silikonhaltiger Emulgatoren,
c) 0,01 - 0,5 Gew.-% eines oder mehrerer Verdicker auf Basis von quervernetzter Polyacrylsäure und Acrylat/$C_{10-30}$ Alkylacrylat Crosspolymer,
d) 1,5 - 6,0 Gew.-% mindestens zweier Ölkomponenten, ausgewählt aus hautpflegenden Kohlenwasserstoffen, Fettalkoholen, Fettsäureestern und deren Mischungen, wobei die Ölkomponenten ausgewählt sind aus den Gruppen besitzend i) schnelles Spreitverhalten, ii) mittleres Spreitverhalten und iii) langsames Spreitverhalten, wobei mindestens zwei Ölkomponenten aus verschiedenen Gruppen i) - iii) ausgewählt sind,
e) 4 - 18 Gew.-% Wasser,
j) 0,01-1,0 Gew.-%, vorzugsweise 0,2-0,6 Gew.-% Co-Verdicker, ausgewählt aus N,N,N',N'-Tetrakis-(hydroxyalkyl)-ethylendiamin, wobei die Hydroxyalkylgruppe vorzugsweise 2-Hydroxypropyl ist, wobei vorzugsweise das Gewichtsverhältnis c) : j) 1:1,5 bis 1:2,5 und bevorzugt 1:2 beträgt.

13. Antimikrobielle Zusammensetzung gemäß einem der vorangehenden Ansprüche, **dadurch gekennzeichnet, dass** die antimikrobielle Zusammensetzung bei 20°C (+/-0,5 °C) eine dynamische Viskosität von 1 bis 12 Pa*s, vzw. 1 bis 5 Pa*s aufweist, gemessen mit einem Brookfield Rotationsviskosimeter Model DV-II+, Spindel Größen 2, 3, 4 oder 5 bei 10 rpm.

14. Antimikrobielle Zusammensetzung gemäß einem der vorangehenden Ansprüche, **dadurch gekennzeichnet, dass** die antimikrobielle Zusammensetzung einen Trübungswert, gemessen als 90° Streulicht-Wert, von mindestens 200 NTU, vorzugsweise mindestens 500 NTU, besonders bevorzugt mindestens 800 NTU aufweist.

15. Antimikrobielle Zusammensetzung gemäß einem der vorangehenden Ansprüche als Desinfektionsmittel, insbesondere zur Desinfektion von belebten Oberflächen, insbesondere zur Händedesinfektion, insbesondere zur hygienischen und chirurgischen Händedesinfektion.

16. Verwendung einer antimikrobiellen Zusammensetzung gemäß einem der Ansprüche 1 bis 14 als Desinfektionsmittel, insbesondere zur Desinfektion von belebten Oberflächen, insbesondere zur Händedesinfektion, insbesondere zur hygienischen und chirurgischen Händedesinfektion.

17. Verfahren zur Herstellung einer antimikrobiellen Zusammensetzung gemäß einem der Ansprüche 1 bis 14, **dadurch gekennzeichnet, dass** zu einer Mischung M1 aus

a1) 8 - 15 Gew.-% Ethanol, 1-Propanol, 2-Propanol oder Mischungen hiervon,

c) 0,1 - 0,3 Gew.-% mindestens eines Verdickers und

e1) 4 - 18 Gew.-% Wasser
eine Mischung M2, bestehend aus
a2) 70 - 80 Gew.-% Ethanol, 1-Propanol, 2-Propanol oder Mischungen hiervon,

j) Co-Verdickern und

d1) mindestens einer in der Mischung aus a2) und j) löslichen Ölkomponente, zugegeben und zur Gelbildung verrührt wird und in diese Gelphase eine Mischung M3 aus

e2) 0 - 14 Gew.-% Wasser, wobei sich e1) und e2) zu 4 - 18 Gew.-% Wasser ergänzen, und

b) 0,1 - 4,0 Gew.-% eines silikonhaltigen Emulgators
und der darin unter Rühren emulgierten

d2) mindestens einen unlöslichen Ölkomponente
eingebracht und unter Rühren zu einer homogenen Zubereitung verarbeitet wird, wobei die Ölkomponenten d1) und d2) ausgewählt sind aus ungesättigen oder gesättigten, linearen oder verzweigten aliphatischen Kohlenwasserstoffen, Fettalkoholen, Fettsäureestern und deren Mischungen.

18. Verfahren zur Herstellung einer antimikrobiellen Zusammensetzung gemäß einem der Ansprüche 1 bis 14, **dadurch gekennzeichnet, dass** zu einer Mischung M4 aus

a) 8 - 15 Gew.-% Ethanol, 1-Propanol, 2-Propanol oder Mischungen hiervon,
c) 0,1 - 0,3 Gew.-% mindestens eines Verdickers
e) 4 - 18 Gew.-% Wasser
eine Mischung M5 aus
b) 0,1 - 4,0 Gew.-% eines silikonhaltigen Emulgators und
d1) 0,5 - 6,0 Gew.-% einer Mischung aus mindestens 3 Ölkomponenten zugegeben und solange gerührt wird, bis sich eine Emulsion bildet, die mit einer Mischung M6 aus
a2) 70 - 80 Gew.-% Ethanol, 1-Propanol, 2-Propanol oder Mischungen hiervon,
j) Co-Verdickern und
d2) mindestens einer in der Mischung aus a2) und j) löslichen Ölkomponente versetzt wird und unter Rühren bis zur Homogenität weiter gerührt wird, wobei die Ölkomponenten d1) und d2) ausgewählt sind aus ungesättigten oder gesättigten, linearen oder verzweigten aliphatischen Kohlenwasserstoffen, Fettalkoholen, Fettsäureestern und deren Mischungen.

**Claims**

1. An antimicrobial compound, containing

a) 80-95 wt.% ethanol, 1-propanol, 2-propanol, or mixtures thereof,
b) one or multiple silicone-containing emulsifiers, wherein the emulsifier is selected from copolymers made from dimethicone with PEG and/or PPG having identical or different chain lengths,
c) one or multiple thickeners based on cross-linked polyacrylic acid or acrylate/$C_{10-30}$ alkyl acrylate cross polymer,
d) at least two oil components, selected from saturated or unsaturated, linear or branched aliphatic hydrocarbons, fatty alcohols, fatty acid esters, and mixtures thereof, wherein the oil components are selected from groups having i) fast spreading behavior, ii) medium spreading behavior, and iii) slow spreading behavior, wherein at least two oil components are selected from different groups i)-iii),
e) water,

**characterized in that** the compound contains soluble oil components up to a proportion of at least 1.5 wt.% and insoluble oil components up to a proportion of a maximum 4.0 wt.%, wherein the total proportion of soluble and insoluble oil components to the antimicrobial compound is at most 6.0 wt.%, at least one oil component is used which is insoluble in the alcohol components, and the silicone-containing emulsifier is selected from the group consisting of cetyl PEG/PPG-10/1 dimethicone, bis-PEG/PPG-20/5 PEG/PPG-20/5 dimethicone, and/or methoxy PEG/PPG-25/4 dimethicone, more preferably 0.1-0.9 wt.% of bis-PEG/PPG-20/5 PEG/PPG-20/5 dimethicone, methoxy PEG/PPG-25/4 dimethicone.

2. The antimicrobial compound according to Claim 1, containing at least one further component selected from

f) pH value regulators,
g) opacifiers,

h) additional antimicrobial active ingredients,
j) co-thickeners.

3. The antimicrobial compound according to Claim 2, **characterized in that** one or multiple co-thickeners j) are used which are selected from the group of amines.

4. The antimicrobial compound according to one of the preceding claims, containing a) 83-89 wt.% ethanol, 1-propanol, 2-propanol, or mixtures thereof, preferably 85-87 wt.% ethanol, 1-propanol, 2-propanol, or mixtures thereof.

5. The antimicrobial compound according to one of the preceding claims, containing b) 0.1-4.0 wt.%, preferably 0.1-2.0 wt.%, particularly preferably 0.1-0.9 wt.% of one or multiple silicone-containing emulsifiers, selected from cetyl PEG/PPG-10/1 dimethicone, bis-PEG/PPG-20/5 PEG/PPG-20/5 dimethicone, and/or methoxy PEG/PPG-25/4 dimethicone, more preferably 0.1-0.9 wt.% of bis-PEG/PPG-20/5 PEG/PPG-20/5 dimethicone, methoxy PEG/PPG-25/4 dimethicone, wherein preferably no other cationic or anionic or amphoteric emulsifiers are included.

6. The antimicrobial compound according to one of the preceding claims, containing c) 0.01-0.5 wt.% of at least one thickener based on cross-linked polyacrylic acid or acrylate/$C_{10-30}$ alkyl acrylate cross polymer, preferably 0.1-0.3 wt.% of at least one thickener based on cross-linked polyacrylic acid or acrylate/$C_{10-30}$ alkyl acrylate cross polymer, and, if necessary j) 0.01-1.0 wt.%, preferably 0.2-0.6 wt.% of a co-thickener, preferably selected from N,N,N',N'-tetrakis (hydroxyalkyl) ethylene diamine, wherein the hydroxyalkyl group is preferably 2-hydroxypropyl, wherein the weight ratio c) : j) is 1:1.5 to 1:2.5, and preferably 1:2.

7. The antimicrobial compound according to one of the preceding claims, containing d) at least three oil components, preferably at least four oil components, more preferably at least five oil components.

8. The antimicrobial compound according to one of the preceding claims, containing at least 1.5 wt.% paraffinum subliquidum, isopropyl palmitate, or mixtures thereof, and at most 4.0 wt.% cetearyl ethylhexanoate, myristyl alcohol, heptamethylnonane, or mixtures thereof, wherein the proportion of soluble and insoluble oil components to the antimicrobial compound is at most 6.0 wt.%.

9. The antimicrobial compound according to one of the preceding claims, containing

f) 0-1.0 wt.% pH regulators, selected from citric acid, lactic acid, tartaric acid, pyroglutamic acid, ascorbic acid, potassium hydroxide, and/or sodium hydroxide,
g) 0-5 wt.% opacifiers, selected from insoluble substances which lead to a cloudy white appearance, preferably selected from powdery polyamide particles or waxy particles in the form of mixtures made of glycol distearate, cocamidopropyl betaine, cocamide monoethanolamine, sodium laureth sulfate, and/or PEG lauryl ether.

10. The antimicrobial compound according to one of the preceding claims, containing h) 0-0.5 wt.% of at least one additional antimicrobial active ingredient, selected from additional antimicrobial active ingredients/substances providing remanence, preferably selected from the group of anionic and nonionic active ingredients, preferably diolen or phenoxyethanol.

11. The antimicrobial compound according to one of the preceding claims, **characterized in that** the antimicrobial compound is present as an emulsion, lotion, gel, or as a hydro-dispersion gel, preferably as an emulsion or hydro-dispersion gel.

12. The antimicrobial compound according to one of the preceding claims, comprising

a) 80-95 wt.% ethanol, 1-propanol, 2-propanol, or mixtures thereof,
b) 0.1-4.0 wt.% of one or multiple silicone-containing emulsifiers,
c) 0.01-0.5 wt.% of one or multiple thickeners based on cross-linked polyacrylic acid or acrylate/$C_{10-30}$ alkyl acrylate cross polymer,
d) 1.5-6.0 wt.% of at least two oil components, selected from hydrocarbons, fatty alcohols, fatty acid esters, and mixtures thereof which are nurturing to the skin, wherein the oil components are selected from groups having i) fast spreading behavior, ii) medium spreading behavior, and iii) slow spreading behavior, wherein at least two oil components are selected from different groups i)-iii),
e) 4-18 wt.% water,

j)0.01-1.0 wt.%, preferably 0.2-0.6 wt.% of a co-thickener, selected from N,N,N',N'-tetrakis (hydroxyalkyl) ethylene diamine, wherein the hydroxyalkyl group is preferably 2-hydroxypropyl,

wherein the weight ratio c) : j) is 1:1.5 to 1:2.5, and preferably 1:2.

13. The antimicrobial compound according to one of the preceding claims, **characterized in that** the antimicrobial compound has a dynamic viscosity of 1 to 12 Pa*s, preferably 1 to 5 Pa*s at 20ºC ($\pm$ 0.5ºC) measured using a Brookfield rotational viscometer, model DV-II+, spindle sizes 2, 3, 4, or 5 at 10 rpm.

14. The antimicrobial compound according to one of the preceding claims, **characterized in that** the antimicrobial compound has an opacity value, measured at a 90º scattered light value, of at least 200 NTU, preferably at least 500 NTU, particularly preferably at least 800 NTU.

15. The antimicrobial compound according to one of the preceding claims as a disinfecting agent, in particular for the disinfection of living surfaces, in particular for hand disinfection, in particular for hygienic and surgical hand disinfection.

16. A use of an antimicrobial compound according to one of Claims 1 through 14 as a disinfecting agent, in particular for the disinfection of living surfaces, in particular for hand disinfection, in particular for hygienic and surgical hand disinfection.

17. A method for producing an antimicrobial compound according to one of Claims 1 through 14, **characterized in that**, a mixture M2 comprising

   a2) 70-80 wt.% ethanol, 1-propanol, 2-propanol, or mixtures thereof,

   j)co-thickeners, and

   d1) at least one oil component soluble in the mixture made of a2) and j),
   is added to a mixture M1 made from
   a1) 8-15 wt.% 1-propanol, 2-propanol, or mixtures thereof,

   c) 0.01-0.3 wt.% of at least one thickener, and

   e1) 4-18 wt.% water
   and stirred to gel formation, and in this gel phase, a mixture M3 is introduced made from
   e2) 0-14. wt.% water, wherein e1) and e2) supplement to 4-18 wt.% water, and

   b) 0.1-4.0 wt.% of a silicone-containing emulsifier, and

   d2) at least one insoluble oil component which is emulsified with stirring,

and processed with stirring to a homogenous preparation, wherein the oil components d1) and d2) are selected from saturated or unsaturated, linear or branched aliphatic hydrocarbons, fatty alcohols, fatty acid esters, and mixtures thereof.

18. The method for producing an antimicrobial compound according to one of Claims 1 through 14, **characterized in that**, a mixture M5 made from

   b) 0.1-4.0 wt.% of a silicone-containing emulsifier, and
   d1) 0.5-6.0 wt.% of a mixture made from at least 3 oil components
   is added to a mixture M4 made from
   a1) 8-15 wt.% ethanol, 1-propanol, 2-propanol, or mixtures thereof,
   c) 0.1-0.3 wt.% of at least one thickener, and
   e1) 4-18 wt.% water
   and stirred until an emulsion forms, which is added to a mixture M6 made from a2) 70-80 wt.% ethanol, 1-propanol, 2-propanol, or mixtures thereof, j)co-thickeners, and
   d2) at least one oil component soluble in the mixture made from a2) and j), and is further stirred with stirring to homogeneity, wherein the oil components d1) and d2) are selected from saturated or unsaturated, linear or branched aliphatic hydrocarbons, fatty alcohols, fatty acid esters, and mixtures thereof.

**Revendications**

1.  Composition antimicrobienne, comprenant :

    a) de 80 à 95 % en poids d'éthanol, de propan-1-ol, de propan-2-ol ou d'un mélange de ceux-ci,
    b) un ou plusieurs émulsifiant(s) contenant une silicone, lequel émulsifiant est choisi parmi les copolymères à base de diméthicone et de PEG et/ou PPG, présentant des chaînes de longueurs égales ou différentes,
    c) un ou plusieurs épaississant(s) à base de poly(acide acrylique) réticulé ou de Cross-polymère acrylate/acrylate d'alkyle en $C_{10-30}$,
    d) au moins deux composants huileux, choisis parmi les hydrocarbures aliphatiques linéaires ou ramifiés, saturés ou insaturés, les alcools gras, les esters d'acide gras et les mélanges de tels composés, étant entendu que ces composants huileux sont choisis dans les groupes de composants

    i) qui s'étalent rapidement
    ii) qui s'étalent moyennement vite,
    iii) et qui s'étalent lentement,

    et que l'on choisit au moins deux composants huileux de groupes (i) à (iii) différents,
    e) et de l'eau,
    **caractérisée en ce que**

    - la composition contient des composants huileux solubles en une proportion d'au moins 1,5 % en poids et des composants huileux insolubles en une proportion d'au plus 4,0 % en poids, la proportion totale des composants huileux solubles et insolubles dans la composition antimicrobienne valant au plus 6,0 % en poids,
    - l'on utilise au moins un composant huileux qui est insoluble dans le composant alcool,
    - et l'émulsifiant contenant une silicone est choisi dans l'ensemble constitué par les cétyl-PEG/PPG-10/1-diméthicone, bis-PEG/PPG-20/5,PEG/PPG-20/5-diméthicone et/ou méthoxy-PEG/PPG-25/4-diméthicone, et de préférence, est constitué de 0,1 à 0,9 % en poids de bis-PEG/PPG-20/S,PEG/PPG-20/5-diméthicone et méthoxy-PEG/PPG-25/4-diméthicone.

2.  Composition antimicrobienne conforme à la revendication 1, qui contient au moins un autre composant choisi parmi :

    f) des agents d'ajustement du pH,
    g) des agents de turbidité,
    h) d'autres substances actives antimicrobiennes,
    j) et des co-épaississants.

3.  Composition antimicrobienne conforme à la revendication 2, **caractérisée en ce que** l'on utilise un ou plusieurs co-épaississant(s) (j), choisi(s) dans l'ensemble des amines.

4.  Composition antimicrobienne conforme à l'une des revendications précédentes, contenant (a) de 83 à 89 % en poids d'éthanol, de propan-1-ol, de propan-2-ol ou d'un mélange de ceux-ci, et de préférence, de 85 à 87 % en poids d'éthanol, de propan-1-ol, de propan-2-ol ou d'un mélange de ceux-ci.

5.  Composition antimicrobienne conforme à l'une des revendications précédentes, contenant (b) de 0,1 à 4,0 % en poids, de préférence de 0,1 à 2,0 % en poids, et mieux encore de 0,1 à 0,9 % en poids d'un ou de plusieurs émulsifiant(s) contenant une silicone, choisi(s) parmi les cétyl-PEG/PPG-10/1-diméthicone, bis-PEG/PPG-20/5,PEG/PPG-20/5-diméthicone et méthoxy-PEG/PPG-25/4-diméthicone, et surtout, constitué(s) de 0,1 à 0,9 % en poids de bis-PEG/PPG-20/5,PEG/PPG-20/5-diméthicone et méthoxy-PEG/PPG-25/4-diméthicone, et qui ne contient de préférence pas d'autres émulsifiants cationiques, anioniques ou amphotères.

6.  Composition antimicrobienne conforme à l'une des revendications précédentes, contenant (c) de 0,01 à 0,5 % en poids d'au moins un épaississant à base de poly(acide acrylique) réticulé ou de Cross-polymère acrylate/acrylate

d'alkyle en $C_{10-30}$, et de préférence de 0,1 à 0,3 % en poids d'au moins un épaississant à base de poly(acide acrylique) réticulé ou de Cross-polymère acrylate/acrylate d'alkyle en $C_{10-30}$, et en option, (j) de 0,01 à 1,0 % en poids, et de préférence de 0,2 à 0,6 % en poids, d'un co-épaississant, choisi de préférence parmi les N,N,N',N'-tétrakis(hydroxy-alkyl)-éthylène-diamines, étant entendu que le groupe hydroxy-alkyle est de préférence le groupe 2-hydroxy-propyle et que le rapport pondéral (c)/(j) vaut de préférence de 1/1,5 à 1/2,5 et mieux encore 1/2.

**7.** Composition antimicrobienne conforme à l'une des revendications précédentes, contenant (d) au moins trois composants huileux, de préférence au moins quatre composants huileux, et mieux encore au moins cinq composants huileux.

**8.** Composition antimicrobienne conforme à l'une des revendications précédentes, qui contient au moins 1,5 % en poids de paraffine huileuse ou pâteuse (paraffinum subliquidum), de palmitate d'isopropyle ou d'un mélange de ceux-ci, et au plus 4,0 % en poids d'éthylhexanoate de cétéaryle, d'alcool myristylique, d'heptaméthyl-nonane ou d'un mélange de ceux-ci, étant entendu que la proportion des composants huileux solubles et insolubles dans la composition antimicrobienne vaut au plus 6,0 % en poids.

**9.** Composition antimicrobienne conforme à l'une des revendications précédentes, qui contient :

f) de 0 à 1,0 % en poids d'agents d'ajustement du pH, choisis parmi de l'acide citrique, de l'acide lactique, de l'acide tartrique, de l'acide pyroglutamique, de l'acide ascorbique, de l'hydroxyde de potassium et/ou de l'hydroxyde de sodium,
g) et de 0 à 5 % en poids d'agents de turbidité, choisis parmi les substances insolubles qui confèrent un aspect trouble, blanchâtre, choisies de préférence parmi des particules poudreuses de polyamide ou des particules cireuses sous forme de mélanges de distéarate de glycol, de cocoamido-propyl-bétaïne, de cocoamidomonoéthanolamine, de lauryl-éther-sulfate de sodium et/ou de PEG-lauryl-éther.

**10.** Composition antimicrobienne conforme à l'une des revendications précédentes, contenant (h) de 0 à 5,0 % en poids d'au moins une autre substance active antimicrobienne choisie parmi les autres substances actives antimicrobiennes/rémanentes, et de préférence, choisie dans l'ensemble des substances actives anioniques ou non-ioniques, surtout les diols ou le phénoxy-éthanol.

**11.** Composition antimicrobienne conforme à l'une des revendications précédentes, **caractérisée en ce que** la composition antimicrobienne se présente sous forme d'émulsion, de lotion, de gel ou de gel-hydrodispersion, et de préférence, sous forme d'émulsion ou de gel-hydrodispersion.

**12.** Composition antimicrobienne conforme à l'une des revendications précédentes, constituée :

a) de 80 à 95 % en poids d'éthanol, de propan-1-ol, de propan-2-ol ou d'un mélange de ceux-ci,
b) de 0,1 à 4,0 % en poids d'un ou de plusieurs émulsifiant(s) contenant une silicone,
c) de 0,01 à 0,5 % en poids d'un ou de plusieurs épaississant(s) à base de poly(acide acrylique) réticulé ou de Cross-polymère acrylate/acrylate d'alkyle en $C_{10-30}$,
d) de 1, 5 à 6,0 % en poids d'au moins deux composants huileux, choisis parmi les hydrocarbures, alcools gras et esters d'acide gras d'entretien de la peau et les mélanges de tels composés, étant entendu que ces composants huileux sont choisis dans les groupes de composants

i) qui s'étalent rapidement
ii) qui s'étalent moyennement vite,
iii) et qui s'étalent lentement,

et que l'on choisit au moins deux composants huileux de groupes (i) à (iii) différents,
e) de 4 à 18 % d'eau,
j) et de 0,01 à 1,0 % en poids, de préférence de 0,2 à 0,6 % en poids, d'un co-épaississant choisi parmi les N,N,N',N'-tétrakis(hydroxyalkyl)-éthylène-diamines, étant entendu que le groupe hydroxyalkyle est de préférence le groupe 2-hydroxy-propyle et que le rapport pondéral (c)/(j) vaut de préférence de 1/1,5 à 1/2,5 et mieux encore 1/2.

**13.** Composition antimicrobienne conforme à l'une des revendications précédentes, **caractérisée en ce que** la composition antimicrobienne présente une viscosité dynamique, mesurée à 20 °C ($\pm$ 0,5 °C) au moyen d'un viscosimètre

EP 2 654 420 B1

rotatif de Brookfield, modèle DV-II+ avec broche de taille 2, 3, 4 ou 5, opérant à 10 t/min, de 1 à 12 Pa.s et de préférence de 1 à 5 Pa.s.

**14.** Composition antimicrobienne conforme à l'une des revendications précédentes, **caractérisée en ce que** la composition antimicrobienne présente un indice de turbidité, mesuré par l'indice de lumière diffusée à 90°, d'au moins 200 UTN (unités de turbidité néphélométrique), de préférence d'au moins 500 UTN, et mieux encore d'au moins 800 UTN.

**15.** Composition antimicrobienne, conforme à l'une des revendications précédentes, en tant qu'agent désinfectant, en particulier pour la désinfection de surfaces vivantes, et plus particulièrement pour la désinfection des mains, notamment la désinfection des mains dans un cadre hygiénique ou chirurgical.

**16.** Utilisation d'une composition antimicrobienne, conforme à l'une des revendications 1 à 14, en tant qu'agent désinfectant, en particulier pour la désinfection de surfaces vivantes, et plus particulièrement pour la désinfection des mains, notamment la désinfection des mains dans un cadre hygiénique ou chirurgical.

**17.** Procédé de préparation d'une composition antimicrobienne conforme à l'une des revendications 1 à 14, **caractérisé en ce qu'**à un mélange M1 constitué

a1) de 8 à 15 % en poids d'éthanol, de propan-1-ol, de propan-2-ol ou d'un mélange de ceux-ci,

c) de 0,1 à 0,3 % en poids d'au moins un épaississant,

e1) et de 4 à 18% d'eau,
on ajoute un mélange M2 constitué
a2) de 70 à 80 % en poids d'éthanol, de propan-1-ol, de propan-2-ol ou d'un mélange de ceux-ci,

j) de co-épaississants,

d1) et d'au moins un composant huileux soluble dans le mélange des constituants (a2) et (j),
et l'on agite le tout jusqu'à la formation d'un gel, on incorpore à cette phase de type gel un mélange M3 constitué
e2) de 0 à 14 % d'eau, étant entendu que les constituants (e1) et (e2) font au total de 4 à 18 % d'eau,

b) et de 0,1 à 4,0 % d'un émulsifiant contenant une silicone,

d2) et du composant huileux insoluble au nombre d'au moins un, qui y a été émulsifié sous agitation,

et l'on travaille le tout sous agitation jusqu'à en faire une préparation homogène, étant entendu que les composants huileux (d1) et (d2) sont choisis parmi les hydrocarbures aliphatiques linéaires ou ramifiés, saturés ou insaturés, les alcools gras, les esters d'acide gras et les mélanges de tels composés.

**18.** Procédé de préparation d'une composition antimicrobienne conforme à l'une des revendications 1 à 14, **caractérisé en ce qu'**à un mélange M4 constitué

a1) de 8 à 15 % en poids d'éthanol, de propan-1-ol, de propan-2-ol ou d'un mélange de ceux-ci,

c) de 0,1 à 0,3 % en poids d'au moins un épaississant,

e1) et de 4 à 18% d'eau,
on ajoute un mélange M5 constitué

b) de 0,1 à 4,0 % d'un émulsifiant contenant une silicone,

d1) et de 0,5 à 6,0 % en poids d'un mélange d'au moins trois composants huileux,
et l'on agite le tout jusqu'à ce qu'il se forme une émulsion, à laquelle on ajoute un mélange M6 constitué
a2) de 70 à 80 % en poids d'éthanol, de propan-1-ol, de propan-2-ol
ou d'un mélange de ceux-ci,

    j) de co-épaississants,

    d2) et d'au moins un composant huileux soluble dans le mélange des constituants (a2) et (j),

et l'on agite le tout jusqu'à homogénéité,
étant entendu que les composants huileux (d1) et (d2) sont choisis parmi les hydrocarbures aliphatiques linéaires ou ramifiés, saturés ou insaturés, les alcools gras, les esters d'acide gras et les mélanges de tels composés.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0930065 B1
- EP 1374847 A1
- WO 2006096239 A1
- US 20040102429 A1
- EP 1281319 A

**In der Beschreibung aufgeführte Nicht-Patentliteratur**

- **W. RAAB ; U. KINDL.** *Pflegekosmetik - Ein Leitfaden, Wissenschaftliche Verlagsgesellschaft mbH Stuttgart,* 2004, 204-205